# EUROPEAN PATENT SPECIFICATION

(11) **EP 3 743 020 B1**
(45) Date of publication and mention of the grant of the patent: **27.03.2024**
(21) Application number: 19702127.2
(22) Date of filing: 24.01.2019
(51) Int. Cl.: A61F 5/00

(54) **IMMOBILISATION ELEMENT WITH PROFILE PROVIDED WITH PROTRUSIONS**
IMMOBILISIERUNGSELEMENT MIT PROFIL MIT VORSPRÜNGEN
ÉLÉMENT D'IMMOBILISATION À PROFILÉ POURVU DE SAILLIES

(30) Priority: 24.01.2018 BE 201805039
(43) Date of publication of application: 02.12.2020
(73) Proprietor: Orfit Industries, 2110 Wijnegem (BE)
(72) Inventor: CUYPERS, Steven, 2970 's Gravenwezel (BE); DE GRUYTERE, Simon, 2450 Meerhout (BE)
(74) Representative: Gevers Patents
(86) International application number: PCT/IB2019/050594
(87) International publication number: WO 2019/145888

(56) References cited:
- WO-A1-98/32402
- WO-A1-2014/193938
- WO-A1-2016/050275
- US-A1- 2007 004 993

## Description

### Technical Field

The present invention concerns an immobilisation element for immobilising a body part of a patient on a supporting surface. More specifically, the invention concerns the anchoring of a profile and a sheet of thermoplastic material of an immobilisation element. The invention also concerns a method for producing the immobilisation element, as defined by the present claims.

### Prior Art

It is known in the prior art that immobilisation elements for immobilising a body part of a patient on a supporting surface are constructed from two essential components, a sheet of thermoplastic material for receiving the body part to be immobilised, and at least one profile for fixing the immobilisation element to the supporting surface.

In the prior art, such immobilisation elements with thermoplastic sheet profile are known, for example from patent applications WO2016050275 and WO9832402, which describes a profile for fixing the immobilisation element to the supporting surface. The profile comprises on a first side a first fixing means for fixing the profile to the sheet, and on a second side different from the first side a second fixing means for releasably fixing the profile to the supporting surface. The first fixing means comprises a groove delimited by a first contact surface and a second contact surface opposite the first contact surface, wherein the groove receives the sheet so that the first and second contact surfaces lie along the sheet. The sheet is connected to the first contact surface and second contact surface by means of application of adhesive between the sheet and the contact surfaces.

In the prior art, the entire immobilisation element, in other words the sheet connected to the profile, is heated such that the sheet of thermoplastic material can easily be plastically deformed. The sheet is then drawn over the patient's body part to be immobilised so that the sheet is plastically deformed in the form of the body part to be immobilised, and after cooling, the thermoplastic material retains its form. The immobilisation element may then be used for immobilising the patient's body part, the shape of which has been assumed by the sheet. For immobilising the body part, the body part is placed on a supporting surface and the immobilisation element is placed over the body part and connected to the supporting surface by means of the profile. The patient's body part must be immobilised on the supporting surface as precisely as possible, and in many applications this immobilisation is subject to repeated positioning of the body part to be immobilised throughout the course of treatment with little variation in the immobilisation position. It is known for example that the success of treatment in proton radiation therapy is already influenced by a deviation of a few millimetres in the positioning of the body part between the simulation and the actual treatment, or between repeated actual treatments. During immobilisation of the body part of the supporting surface, tensile forces occur between the sheet and the profile both during the initial drawing of the sheet over the body part to be immobilised, and on movement of the patient and consequently of the body part to be immobilised. The immobilisation element from the prior art is not however resistant to these tensile forces, partly because of a poor connection between the sheet and the profile and because of the environmental influences on the bonding strength of the adhesive, such as the influence of the higher temperature during the initial deformation of the sheet, the degradation of the adhesive over time, for example because of moisture absorption in damp storage areas, and/or for example by atmospheric influences such as varying temperatures, and/or for example degradation of the adhesive due to soiled surfaces during the gluing process. The result is that in these immobilisation elements, the sheet is subjected to high loads and hence for example becomes detached from the profile during use. In many applications of immobilisation elements, this consequence is harmful since the ability to immobilise a body part repeatedly in virtually the same position is often crucial.

### Description of the Invention

An object of the present invention is to provide a method for producing an immobilisation element with which the above-mentioned problems are remedied. More specifically, the object of the invention is to provide a method for producing an immobilisation element which has a better connection under the influence of tensile forces which occur between the sheet and the profile of the immobilisation element. For this, a method comprises the following steps:
- providing a sheet of thermoplastic material for receiving the body part to be immobilised,
- providing a profile which on a first side comprises a first fixing means for fixing the profile to the sheet, and on a second side different from the first side comprises a second fixing means for releasably fixing the profile to the supporting surface, wherein the first fixing means comprises a groove delimited by a first contact surface and a second contact surface opposite the first contact surface for receiving the sheet of thermoplastic material,
- placing the sheet of thermoplastic material in the groove of the first fixing means so that the first and the second contact surfaces lie along the sheet,
- creating at least one protrusion in at least one of the first contact surface and the second contact surface so that the protrusion penetrates the sheet of thermoplastic material in order to anchor the sheet of thermoplastic material in the groove as defined by the appended claims.

The present invention has the advantage that by anchoring the sheet of thermoplastic material in the groove of the profile by creating at least one protrusion provided in at least one of the first and second contact surfaces, the sheet of thermoplastic material is attached to the profile in a stable and firm fashion. The method for example provides a profile which attaches the sheet firmly and immovably for a long period. The immobilisation element obtained according to the method of the present invention is resistant to the tensile forces which occur between the sheet and the profile both during the initial pulling of the sheet over the body part to be immobilised and on movement of the patient during the period of treatments. The movement of the patient and consequently the body part to be immobilised during immobilisation of the body part however creates high tensile forces between the profile and the anchored sheet, which tensile forces try to pull the sheet out of the profile. In the immobilisation element obtained according to the method of the present invention, the risk is reduced that the sheet in the groove of the profile will tear, deform or become detached under the influence of the tensile forces occurring. The immobilisation element obtained according to the method of the present invention therefore has the advantage that the body part to be immobilised can be positioned and immobilised on the supporting surface in a reliable and precise fashion. The body part may for example be positioned and immobilised repeatedly during the course of the treatment with little variation in the immobilisation position. A secondary advantage of the present invention is that the anchoring of the sheet in the groove of the profile is not affected by environmental factors such as a damp environment, whereby the anchoring remains stable for a long period.

A secondary advantage of the present invention is that no auxiliary material need be used for clamping the sheet in the groove of the profile, whereby costs are saved which for example would be necessary for procuring anchoring elements such as a screw. In addition, the immobilisation element obtained according to the method of the present invention is not influenced in use by secondary fixing materials which could affect the function of the immobilisation element. Thus for example the at least one protrusion exerts virtually no influence on electromagnetic radiation such as X-ray, gamma ray, electron and proton beams, and also ultrasonic waves which for example are used during a radiation therapy or during a medical diagnosis. This effect is for example the result of the homogeneous material properties of the at least one protrusion and the other parts of the profile, which for example are both made of the same material and consequently constructed from the same particles with the same atomic mass. The method according to the present invention offers a secondary advantage that clamping of the sheet by means of the at least one protrusion is a time-saving and cheap method for producing the immobilisation element, since no separate fixing elements need be provided such as the application of an adhesive, wherein the adhesive must harden before use of the immobilisation element, or such as anchoring of the sheet in the groove by means of ultrasound welds, for which costly equipment must be procured. A secondary advantage of the present invention is that the immobilisation element may be produced just before use of the immobilisation element, since after the sheet has been clamped in the groove of the profile by means of the at least one protrusion, the immobilisation element can be used directly, for example in contrast to anchoring the sheet in the groove by means of adhesive, wherein the use of the immobilisation element must wait until the adhesive has set. As a result, it is possible to produce the immobilisation element on site, for example just before use of the immobilisation element for immobilising a patient's body part on a supporting surface. Consequently, the profiles and the sheets of thermoplastic material may be stored separately from each other by the user of the immobilisation element, which for example leads to more compact packaging and storage. The more compact packaging and storage means for example that the production and usage process becomes more environmentally friendly and economically advantageous, for example because the immobilisation elements can be transported more efficiently and for example since less packaging must be used. In addition, this means that the user of the immobilisation element may store separately on site firstly a stock of sheets of various sizes, such as in child format or obese format, and various types such as immobilisation masks or arm splints, and secondly a stock of profiles in different types, for example depending on the type of supporting surface. The user may then decide on site which type of sheet should be anchored to which type of profile, depending on the patient and the body part to be immobilised, which relieves the user of the need to procure the precise combination of sheet and profile in advance.

In one embodiment of the present invention, the steps contained in the method for producing the immobilisation element are successive steps. This has the advantage that the protrusions are created when the sheet is placed in the groove of the profile, whereby a better anchoring of the sheet in the groove is obtained. More specifically, in this embodiment the profile may be made of a rigid material, but it is not necessary for the contact surfaces to have to be pulled apart for placing the sheet in the groove, which would be necessary if the contact surfaces were already provided with protrusions. The firm connection between the sheet and the profile is partly the result of forming a rivet connection using the protrusion. The inventors have found that creating at least one protrusion in at least one contact surface by deep-drawing of a small area of the contact surface leads to a protrusion with thin protrusion walls and a relatively thick protrusion head. The protrusion thus created has a relatively hard and rigid protrusion head, compared with relatively flexible protrusion walls. The sheet of thermoplastic material is elastically stretched by penetration of the protrusion, whereby it has a tendency to spring back resiliently. Because the protrusion walls are relatively flexible in comparison with the protrusion head, the sheet springs back resiliently by compression of the protrusion walls. Thus at least part of the sheet is pinched between the protrusion head and the contact surface, thus forming a rivet connection.

According to the method of the present invention, the step of creating at least one protrusion in at least one of the first contact surface and second contact surface is carried out by pressing together a die and the profile, for example by pressing the die around the profile, so that the profile is plastically deformed until the first and second contact surfaces are provided with at least one protrusion. In one embodiment of the method of the present invention, the die is provided with teeth or pins which press the at least one protrusion into the profile. The embodiment in which the protrusion is created by compressing a die and the profile offers the advantage that a reproducible result can be obtained because each profile is compressed with the same die in the same way.

Furthermore, the present invention provides an immobilisation element wherein at least one of the first contact surface and the second contact surface of the profile is provided with at least one protrusion, wherein the protrusion penetrates the sheet of thermoplastic material in the thickness direction of the sheet in order to anchor the sheet of thermoplastic material in the groove. The present invention provides an immobilisation element obtained by performing the present method. The present invention has the advantage that by anchoring the sheet of thermoplastic material in the groove of the profile by means of at least one protrusion provided in at least one of the first and second contact surface, the sheet of thermoplastic material is attached to the profile in a stable and firm fashion for a long period. The profile is for example resistant to the separation of the sheet from the groove of the profile.

The sheet of thermoplastic material is anchored in the groove mainly by clamping of the sheet by the at least one protrusion. This embodiment illustrates that no secondary fixing means need be used for anchoring the sheet in the groove of the profile. Since the anchoring takes place substantially by clamping the sheet in the groove by means of the protrusion which penetrates the sheet, no secondary materials need be used which would increase costs and production time and hinder usage. The term "penetration" of the protrusion into the sheet means that the protrusion creates an indentation in the sheet relative to the point where no protrusion is provided, whereby for example the frictional resistance between the sheet and the contact surface is increased, and/or whereby for example the sheet is clipped over the protrusion, and/or whereby for example the protrusion forms a rivet connection.

Described is a groove that has an elongate direction which is referred to below as the longitudinal direction of the groove, a shorter transverse direction approximately perpendicular to the contact surfaces, and a depth direction perpendicular to the longitudinal and transverse directions of the groove. The width of the groove measured in the transverse direction of the groove is approximately constant over the entire groove. The width of the groove measured in the transverse direction of the groove is between 1 mm and 5 mm. The sheet of thermoplastic material in the groove has a thickness d1 in the thickness direction of the sheet. More specifically, the sheet in the groove has a thickness d1 where no protrusions are provided in the sheet. More specifically, the sheet in the groove has a thickness d1 before deformation of the sheet over the body part to be immobilised. The thickness d1 of the sheet is between 0.5 mm and 5.0 mm. The width of the groove is greater than the thickness d1 of the sheet inside the groove. This has the advantage that the thermoplastic sheet can be mounted simply, rapidly and manually during production. The width of the groove can be equal to the thickness d1 of the sheet inside the groove, for example because the contact surfaces of the groove compress the sheet, whereby the thickness of the sheet outside the groove is greater than or equal to the thickness d1 of the sheet inside the groove. The advantage of this embodiment is that the sheet of thermoplastic material is more firmly anchored in the groove, for example by friction between the contact surfaces and the sheet resulting from clamping of the sheet by the protrusion.

The groove of the profile can be delimited by a first wall and an opposite second wall, and the first and second contact surfaces form part of the first and second walls respectively, wherein the contact surface is the part of the wall which lies along the sheet of thermoplastic material. The first wall and the second wall can be integrally joined together, for example because the first wall and the second wall were formed from the same element, for example by folding a plate or by an injection-moulding process or an extrusion process. Alternatively, the first wall and the second wall of the groove are not integrally joined together, for example one of the two walls is releasably connected to the other wall of the groove. Provision of the two walls integrally connected to each other offers the advantage that the groove of the profile consists of one piece, which avoids weak points in the profile and for example reduces the risk of undesirable separation of the two walls during use of the immobilisation element. The provision of the two releasable walls offers the advantage that the sheet can easily be extracted from the groove, which for example simplifies recycling of the sheet or the profile after use of the immobilisation element. Alternatively, the thickness of the walls is between 1 mm and 5 mm. The provision of the walls with the given thickness offers the advantage that during creation of the protrusion, for example during deep-drawing of the protrusion, the protrusion walls become thinner and more flexible in order to be able to form a rivet connection. The provision of thicker walls initially leads to protrusions with relatively rigid protrusion walls which make it difficult to form a rivet connection, for example since the sheet does not spring back sufficiently strongly to press the rigid protrusion walls substantially inward. The provision of thinner walls initially leads to a protrusion with protrusion walls which are too flexible, which may for example lead to a less precise rivet connection, for example because under the influence of tensile forces, the protrusion walls can move over a limited distance in the direction of the tensile force. Alternatively, the thickness of the protrusion wall is adapted to the thickness of the sheet; preferably the thickness of the protrusion walls is proportional to the thickness of the sheet so that, after thinning of the protrusion walls by deep-drawing of the protrusion, sufficiently thick and consequently rigid protrusion walls are obtained. Alternatively, the thickness of the walls is 2 mm if the thickness d1 of the sheet is 2 mm, and a thicker wall is provided if a thicker sheet is provided. The provision of thicker walls on provision of a thicker sheet offers the advantage that, on creation of the protrusion by deep-drawing of the wall, the protrusion walls are given sufficient but limited flexibility in order to be able to form a firm rivet connection. The provision of a thicker sheet initially however requires the wall to be drawn more deeply, and consequently leads to thinner and less rigid protrusion walls.

Alternatively, the protrusion protrudes by a maximal height h1 from the contact surface on which the protrusion is provided. In one embodiment, the height h1 is smaller than the thickness d1 of the sheet. Because the protrusion protrudes from the contact surface by less than the thickness of the sheet, the protrusion cannot bore through the sheet. More specifically, this embodiment provides at least one protrusion which penetrates, but only partially penetrates, the sheet of thermoplastic material in the thickness direction of the sheet. This has the advantage that tensile forces are supported by a larger area of thermoplastic material. This has the advantage that the risk of tearing or undesirable deformation of the sheet in the groove of the profile is reduced. In an alternative embodiment, the height h1 of the protrusion is greater than the thickness d1 of the sheet, but the protrusion only penetrates the sheet partially. This is achieved for example because the contact surface opposite the contact surface on which the protrusion is provided deforms during creation of the protrusion, and because the sheet deforms into a fleece which is clamped between the protrusion and the opposite contact surface. Preferably, the method is provided for creating the protrusions with height h1. Preferably, the method for producing the immobilisation element provides a die, the teeth of which have a corresponding height greater than h1, more specifically at least as large as the sum of the height h1 and the thickness of the wall of the groove of the profile. Alternatively, the ratio between the maximum height h1 of the protrusion relative to the thickness d1 of the sheet is between 0.5 and 1.25, more preferably between 0.8 and 1.25. The inventors have found that with these ratios, an optimal stability is achieved in anchoring the sheet in the groove. Immobilisation elements with a higher ratio have a higher risk of tearing and undesirable deformation of the sheet, while immobilisation elements with a lower ratio have a higher risk of detachment of the sheet from the groove of the profile. Preferably, the method is provided for creating protrusions with height h1.

Alternatively, at least one of the first and second contact surfaces has at least one protrusion which penetrates, but only partially penetrates, the sheet of thermoplastic material in the thickness direction of the sheet, in order to provide a fleece of thermoplastic material between the protrusion and the contact surface. Alternatively, the fleece has a minimal thickness without being punctured by the protrusion. Preferably, the ratio of the minimal thickness of the fleece to the thickness d1 of the sheet is between 0.0001 and 0.5, preferably between 0.0001 and 0.2. Preferably, the ratio of the minimal thickness of the fleece to the width of the groove is between 0.0001 and 0.5, preferably between 0.0001 and 0.2. Preferably, the method is provided for creating protrusions which penetrate, but only partially penetrate, the sheet of thermoplastic material. Preferably, the method is provided for creating the fleece of thermoplastic material. The advantage of this embodiment is that the sheet of thermoplastic material deforms under the influence of the pressure force into a fleece of thermoplastic material between the protrusion and the opposite contact surface, which achieves a better clamping of the sheet in the groove. Without being restricted to one theory, the better clamping is a consequence for example of the flowing of the sheet of thermoplastic material under the influence of the increased pressure force, whereby after elimination of the pressure force, the sheet of thermoplastic material forms a stable adhesion layer with a high level of friction between the protrusion and the opposite contact surface, which anchors the sheet firmly in the groove. In addition, the great deformation of the sheet ensures the interruption of a non-stick layer, which is applied on the sheets of thermoplastic material in order for example to prevent them sticking to each other during storage, whereby the adhesive properties of the fleece increase.

Alternatively, the contact surface opposite the contact surface on which the at least one protrusion is provided, opposite the at least one protrusion, has at least one bulge, wherein the bulge points away from the sheet. More specifically, the bulge is oriented in the same direction as the opposite protrusion. Alternatively, the protrusion and the bulge have almost symmetrical, for example congruent forms. Alternatively, the height h1 of the protrusion is smaller than the width of the groove. In an alternative embodiment, the height h1 of the protrusion is greater than the width of the groove, and at least one fleece of the sheet is provided between the protrusion and the bulge. More generally, this embodiment provides at least one protrusion which penetrates, but only partially penetrates, the sheet of thermoplastic material in the thickness direction of the sheet, in order to provide a fleece of thermoplastic material between the protrusion and the bulge. Preferably, the method is provided for creating the bulge, for example by providing a die with teeth of appropriate height, or providing an adjustable depth setting. More specifically, the method is provided for creating the bulge during creation of the protrusion, for example by the plastic deformation of the contact surface opposite the contact surface provided with the protrusions during the compression of the die with the profile. More specifically, during compression of the die with the profile, the pressure force is transferred from the first contact surface on which the protrusion is provided to the opposite contact surface via the sheet of thermoplastic material. The advantage of this embodiment is that the sheet of thermoplastic material deforms under the influence of the pressure force into a fleece of thermoplastic material between the protrusion and the bulge, which achieves a better clamping of the sheet in the groove. Without wanting to be restricted to one theory, the better clamping is a result for example of flowing of the sheet of thermoplastic material under the influence of the increased pressure force, whereby after elimination of the pressure force, the sheet of thermoplastic material forms a stable adhesive layer between the protrusion and the bulge, which anchors the sheet firmly in the groove. The advantage of this embodiment is furthermore that the user of the immobilisation element or the manufacturer of the immobilisation element can immediately establish visually whether or not the profile is provided with protrusions, or can establish whether all protrusions have been correctly applied, for example by visually establishing the presence of bulges on the contact surface. A secondary advantage is that the sheet is clamped more firmly in the groove of the profile.

Alternatively, the cross-section of the protrusion, following a plane parallel to the contact surface on which the protrusion is provided, has a transverse cross-section mainly in one of the following forms: a rectangle, an oval, a circle, a triangle, a polygon. In one embodiment, the plane in which the cross-section is formed lies in the contact surface on which the protrusion is provided. In a further embodiment, the plane with which the cross-section is formed lies both inside the protrusion and inside the sheet of thermoplastic material. The immobilisation element in which the form of the transverse cross-section of the protrusion is a circle offers the advantage that the protrusion has no corners from which the sheet of thermoplastic material can begin to tear under the influence of tensile stress between the sheet and the protrusion. Alternatively, the transverse cross-section of the at least one protrusion, together with the contact surface, describes approximately a circle with a diameter of between 0.5 mm and 5 mm. This embodiment offers the advantage of limiting the surface area of the contact surface on which the teeth of the die press in order to create the protrusion, whereby the protrusions can be created with limited pressure force. In one embodiment of the present invention, the teeth of the die are approximately cylindrical in shape and have a circular transverse cross-section with a diameter of between 0.5 mm and 5 mm. In an alternative embodiment of the present invention, the transverse cross-section of the protrusion has substantially a rectangular form, mainly oriented along the longitudinal direction of the groove, and the protrusion extends approximately over the entire length of the contact surface. This embodiment offers the advantage of guaranteeing a firm anchoring of the sheet in the groove. The choice of the form of transverse cross-section may be selected by the person skilled in the art, amongst others depending on the required grip of the protrusion on the sheet of thermoplastic material, the risk of tearing of the sheet on any sharp edges of the protrusion, and the deformability of the profile material. Preferably, the method is provided for creating the form of the cross-sections of the protrusions. Preferably, the method for producing the immobilisation element provides for this a die, the teeth of which have a corresponding form.

Alternatively, following a plane perpendicular to the contact surface on which the protrusion is provided, the protrusion has a longitudinal cross-section mainly in one of the following forms: a rectangle, at least part of an oval, at least part of a circle, a triangle, a trapezium and a rectangle provided with a triangular notch in the side opposite the side in the contact surface. Alternatively, the longitudinal cross-section of the protrusion has a form consisting of a rectangle provided with a triangle or a semicircle on top of the side opposite the side in the contact surface. The choice of the form of longitudinal cross-section may be selected by the person skilled in the art, amongst others depending on the required grip of the protrusion in the sheet of thermoplastic material, the risk of tearing of the sheet on any sharp edges of the protrusion, and the deformability of the profile material. Preferably, the method is provided for creating the form of the cross-sections of the protrusions.

Alternatively, the protrusion has one of the following three-dimensional forms: a half bowl, a bar, a pyramid, a cone, a cylinder provided with a conical notch in the surface opposite the contact surface, a cylinder with partially or fully rounded ends. The choice of the three-dimensional form of the protrusion may be selected by the person skilled in the art, amongst others depending on the required grip of the protrusion in the sheet of thermoplastic material, the risk of tearing of the sheet on any sharp edges of the protrusion, and the deformability of the profile material. Preferably, the method is provided for creating the form of the cross-sections of the protrusions. Preferably, the method for producing the immobilisation element provides for this a die, the teeth of which have a corresponding form.

Alternatively, the protrusion is at least partially hollow, for example substantially hollow, for example only provided with edge faces which delimit the protrusion. The provision of a hollow protrusion in comparison with a solid protrusion offers the advantage that the weight of the profile may be reduced. A secondary advantage is that the profile provided with a hollow protrusion, compared with a solid protrusion, exerts less influence on radiation such as X-rays, gamma rays, electron beams, proton beams and ultrasonic waves which are used for example during a radiation therapy or during a medical diagnosis; for example, because less material is present in the profile which absorbs, reflects and deflects these rays.

Alternatively, the protrusion is hollow and not provided with an edge face in the contact surface. The advantage of this embodiment is that the user of the immobilisation element or the manufacturer of the immobilisation element can immediately establish visually whether or not the profile is provided with protrusions, or can establish whether all protrusions are applied correctly, which is more difficult to establish from the groove as this is often not wide enough to inspect easily, or if the groove is already provided with the sheet. In addition, a hollow protrusion is easier to make than a solid protrusion, for example by pressing the protrusion, which says production costs and time. Preferably, the method is provided for creating hollow protrusions, for example by the plastic deformation of the contact surface.

In one embodiment of the present invention, the at least one protrusion consists of the same material as the contact surface on which the at least one protrusion is provided. This embodiment has the advantage that the protrusion exerts virtually no influence on radiation such as X-rays, gamma rays, electron beams, proton beams and ultrasonic waves which are used for example during a radiation therapy or during a medical diagnosis; for example, because of the homogeneous material properties of the at least one protrusion and the other parts of the profile, which for example are both made of the same material and consequently constructed from the same particles with the same atomic mass. Preferably, the method of the present invention is provided for creating at least one protrusion from the same material as the contact surface by compressing the contact surface with a die provided with teeth, in order to plastically deform the contact surface into a protrusion.

In one embodiment of the present invention, at least one protrusion is integral with the contact surface, for example connected to the contact surface. This embodiment emphasises that the protrusion is formed by the plastic deformation of the contact surface, whereby the contact surface and protrusion are integral. This offers the advantage of reducing the risk that the protrusion is pulled away together with the sheet under the influence of the tensile forces between the profile and the sheet.

In one embodiment of the present invention, at least one of the first contact surface and the second contact surface is provided with a set of protrusions. The provision of a set of protrusions offers the advantage that the sheet is anchored better, for example in a more stable and stronger fashion, in the groove of the profile. Preferably, the method is provided for creating a set of protrusions on at least one of the contact surfaces, for example by providing a set of teeth on the die.

Alternatively, the set of protrusions comprises at least one row of protrusions, wherein the focal points of the cross-sections of the protrusions in the at least one row and the contact surface on which the protrusions are provided lie substantially on the same straight line, preferably oriented along the longitudinal direction of the groove. The direction of the straight line on which the protrusions of the row are provided is called the longitudinal direction of the row. The provision of a row of protrusions offers the advantage that the sheet is clamped at various locations, which achieves a better anchoring. The provision of a row of protrusions oriented along the longitudinal direction of the groove offers the advantage that tensile forces on the sheet are absorbed by several protrusions, which leads to a more homogeneous spread and reduction of the tensile stress over a greater surface area, and hence reduces the risk of tearing or detachment or undesirable deformation of the sheet. In one embodiment of the present invention, the set of protrusions comprises a first row of protrusions and at least one following second row of protrusions. Preferably, the first row of protrusions and the at least one following second row of protrusions run parallel to each other. The provision of at least two parallel rows of protrusions offers the advantage that the sheet is anchored better in the groove of the profile, for example because the friction between the sheet and the profile increases. Preferably, the method is provided for creating at least one row of protrusions. Preferably, the method for production of the immobilisation element provides for this at least one die provided with at least one row of teeth.

Alternatively, the focal point of the cross-section of each protrusion provided in the at least one row of protrusions on the contact surface on which the at least one row of protrusions are provided, referred to below as the transverse cross-section of the protrusion in the contact surface, is remote by an inter-protrusion distance from the focal point of the at least one neighbouring protrusion in the row of protrusions. Alternatively, the inter-protrusion distances of each protrusion in the at least one row are approximately equal, thus forming a regular row. The provision of at least one regular row, for example oriented along the longitudinal direction of the groove, offers the advantage that tensile forces on the sheet are absorbed by several protrusions and distributed regularly, whereby the tensile stress is distributed more homogeneously over a greater area and hence the risk of tearing or detachment or undesirable deformation of the sheet is reduced. Preferably, the method is provided for creating at least one regular row of protrusions.

Alternatively, the first row of protrusions and the at least one following second row of protrusions of the set of protrusions are regular rows with the same inter-protrusion distance. Alternatively, the two regular rows run parallel to each other. Alternatively, the at least two regular rows are offset relative to each other along the longitudinal direction of the row by a distance which is smaller than the inter-protrusion distance of the row. The provision of at least two regular rows offers the advantage that the sheet can be anchored better in the groove of the profile. In addition, the first regular row of protrusions and the at least one following second regular row of protrusions penetrate the sheet of thermoplastic material alternately in the longitudinal direction of the contact surface, which achieves a more homogeneous spread of the tensile stress in the sheet. Preferably, the method is provided for creating two offset regular rows of protrusions, for example by providing corresponding teeth on the die.

In one embodiment of the present invention, both the first contact surface and the second contact surface are provided with at least one protrusion. The provision of at least one protrusion on both the first contact surface and on the second contact surface offers the advantage that the sheet is anchored better in the groove of the profile. Preferably, the method is provided for creating the protrusions on both contact surfaces. Preferably, the method for production of the immobilisation element provides for this a die device equipped with opposing dies provided with teeth.

In one embodiment of the present invention, both the first contact surface and the second contact surface are provided with a set of protrusions. This offers the advantage that the sheet is anchored better in the groove of the profile. In addition, this offers the advantage that there is more chance of the profile having a straight form after formation of the protrusions, since both contact surfaces are deformed for forming the protrusions. Preferably, the method is provided for creating the sets of protrusions on both contact surfaces, for example by providing corresponding sets of teeth on both dies of the die device.

In one embodiment of the present invention, at least some of the protrusions of the set of protrusions provided on the first contact surface and some of the protrusions of the set of protrusions provided on the second contact surface lie in the extension of each other and thus form overlapping protrusions. This has the advantage that during creation of the protrusions, the sheet of thermoplastic material is compressed between the overlapping protrusions, which ensures a better adhesion, for example a higher friction, between the protrusions and the sheet of thermoplastic material. The compression may for example lead to the local heating and plasticisation of the sheet of thermoplastic material between the overlapping protrusions, whereby the material encapsulates the protrusion more easily and binds the latter, for example on cooling and hardening of the thermoplastic material. Preferably, the method is provided for creating overlapping protrusions on both contact surfaces, for example by providing overlapping teeth on both dies of the die device. This has the secondary advantage that the protrusions can be created in a simple fashion because compression of the dies with the contact surfaces does not lead to the folding of the sheet of thermoplastic material between non-overlapping protrusions. In one embodiment of the present invention, the sheet of thermoplastic material in the groove has a thickness d1 in the thickness direction of the sheet, and the overlapping protrusions of the first contact surface and the second contact surface protrude by a respective maximal height h2 and h3 relative to the contact surface on which the protrusions are provided, and the height h2 + h3 is smaller than the thickness d1. More specifically, the sheet in the groove has a thickness d1 at the point where no protrusions are provided in the sheet. Because the overlapping protrusions together protrude from the contact surface by less than the thickness of the sheet, the protrusion cannot bore through the sheet. This has the advantage that tensile forces are absorbed by a greater area of thermoplastic material. This has the advantage that the risk of tearing or undesirable deformation or detachment of the sheet in the groove of the profile is reduced. Preferably, the method is provided for creating overlapping protrusions with respective maximal heights h2 and h3. In one embodiment of the present invention, the ratio between the height h2 + h3 of the overlapping protrusions of the set of protrusions, relative to the thickness d1 of the sheet of thermoplastic material, lies between 0.5 and 0.999, preferably between 0.8 and 0.999. The inventors have found that this ratio achieves an optimal anchoring of the sheet in the groove. A higher ratio results in less risk of the sheet detaching from the groove, partly because of the greater frictional resistance between the sheet and the groove of the profile, but in return increases the risk of deformation and tearing of the sheet, partly because of the smaller residual cross-sectional area which remains in the sheet in the extension of the protrusion. A lower ratio results in a lower risk of tearing and deformation of the sheet, but in return increases the risk of detachment of the sheet from the groove of the profile. Preferably, the method is provided for creating overlapping protrusions with the specified ratio between heights.

Alternatively, at least some of the protrusions, for example all the protrusions, which are provided on the first contact surface, and at least some of the protrusions, for example all the protrusions, which are provided on the second contact surface are not overlapping protrusions, for example are non-overlapping protrusions. This has the advantage that the protrusions of the set of protrusions provided on the first and second contact surfaces can form bulges on the respective second and first opposite contact surfaces. Preferably, the method is provided for creating non-overlapping protrusions, for example by the provision of non-overlapping teeth on two opposing dies.

Alternatively, both the set of protrusions provided on the first contact surface and the set of protrusions provided on the second contact surface form at least one regular row. In one embodiment of the present invention, the at least one regular row on the first contact surface and on the second contact surface have the same inter-protrusion distance. Alternatively, the at least one regular row of protrusions provided on the first contact surface and the at least one regular row of protrusions provided on the second contact surface run parallel to each other, for example in the longitudinal direction of the groove. Alternatively, the at least one regular row of protrusions of the set of protrusions provided on the first contact surface and the at least one regular row of protrusions of the set of protrusions provided on the second contact surface are offset relative to each other along the longitudinal direction of the row by a distance which is smaller than the inter-protrusion distance of the row. The advantage of these embodiments is that a more homogeneous spread of the tensile stress can be obtained in the sheet of thermoplastic material, which avoids deformation or tearing as a result of excessive stress concentrations. Preferably, the method is provided for creating at least one regular row of protrusions on both contact surfaces, for example by providing corresponding regular rows of teeth on both dies of the die device.

Alternatively, the sheet of thermoplastic material for receiving the body part to be immobilised is selected from the group comprising thermoplastic elastomer, thermoplastic polyurethane, thermoplastic polyisoprene, thermoplastic polyester, thermoplastic polyolefins, polyvinyl chloride, polystyrene, or a mixture of two or more of these materials. Alternatively, the sheet of thermoplastic material for receiving the body part to be immobilised has a deformation temperature of between 65°C and 70°C, wherein the deformation temperature is a temperature at which the sheet is drawn over the body part to be immobilised such that after the sheet of thermoplastic material has cooled, this retains the form of the body part to be immobilised. The advantage of the materials of these embodiments is that they are easily deformable at a deformation temperature which can be tolerated by the patient's skin, such that the initial deformation of the sheet by stretching the sheet over the body part of the patient to be immobilised can take place as comfortably as possible. In addition, these materials offer the desired elastic spring force of the sheet on creation of the protrusion, for example in order to form a rivet connection and/or in order to form a fleece of thermoplastic material which pinches around the protrusion.

Alternatively, the at least one protrusion clamps the sheet at a deformation temperature with a clamping force of at least 100N per profile. Alternatively, the at least one protrusion clamps the sheet at room temperature, more specifically at the temperature at which the immobilisation element is used for example during treatment, more specifically at around 21°C, with a clamping force of at least 130N per profile. The inventors have found that with this clamping force, the immobilisation element is not damaged, for example is not damaged by tearing of the sheet, nor by detachment of the sheet from the groove, nor by the undesirable plastic deformation of the sheet. The inventors have furthermore found that this clamping force at deformation temperature is sufficient, in the most common usage situations, to resist the clamping forces which occur during the initial deformation of the sheet of plastic material in order to assume the form of the body part to be immobilised. The inventors have furthermore found that this clamping force at room temperature is sufficient, in the most common usage situations, to resist the clamping forces which occur during actual use of the immobilisation element, for example during treatment of the patient.

The sheet of thermoplastic material comprises a single sheet.

Alternatively, the sheet of thermoplastic material comprises several sheets bonded to each other to form a structural whole. In one embodiment of the present invention, the sheet of thermoplastic material has homogeneous material properties throughout the sheet, such as a homogeneous modulus of elasticity, homogeneous melt temperature and homogeneous toughness. In an alternative embodiment of the present invention, the sheet of thermoplastic material has heterogeneous material properties, for example heterogeneous throughout the thickness of the sheet or heterogeneous over the area of the sheet. The sheet of thermoplastic material may for example be composed of several sheets with different material properties, or may be hardened in different amounts, for example by different parts of the sheet being exposed to different UV treatment parameters.

Alternatively, the sheet of thermoplastic material is perforated, for example to allow evaporation via the skin and/or to allow visual inspection of the skin under the sheet, and/or to reduce attenuation of the sheet to a minimum. Preferably, the part of the sheet of thermoplastic material in the groove of the profile is not perforated, in order to reduce the risk of deformation or tearing of the sheet in the groove. Preferably, the method is provided for perforating the sheet of thermoplastic material. Preferably, the method provides perforation of the sheet for clamping of the sheet in the groove of the profile.

Alternatively, the sheet of thermoplastic material is anchored permanently in the groove of the profile, more specifically so as to be difficult to release. This offers the advantage that once the immobilisation element has been assembled, the sheet can no longer easily be detached from the profile. This reduces the risk that the sheet may be accidentally removed from the profile by a user of the immobilisation element, which for example may be problematical if the same immobilisation element must be reused for the same patient in a succession of treatments.

Alternatively, the profile on a first side comprises a first fixing means for fixing the profile to the sheet, and on a second side different from the first side, a second fixing means for releasably fixing the profile to the supporting surface. In one embodiment of the present invention, the first and second fixing means are provided on opposite ends of the profile. Alternatively, the second fixing means is adapted to the type of supporting surface to which the profile is releasably connected. Examples of different types of second fixing means can be found in patent publications WO9832402 and WO2016050275.

Alternatively, the profile is made from a plastic material selected from the group of POM (polyoxymethylene), ABS (acrylonitrile butadiene styrene), PVC (polyvinyl chloride), polystyrene, polyamide, polypropylene, polycarbonate, (thermoplastic) polyurethane or an equivalent material. Preferably, the material of the profile has a higher impact hardness than the sheet, for example a higher Mohs hardness. Preferably, the profile has a higher indentation hardness than the sheet, for example a higher Vickers hardness or Rockwell hardness. The provision of the profile from these materials has the advantage that the protrusion provided in the profile is easier to attach, that the profile retains its stiffness during heating of the sheet, and that the risk of the sheet damaging the protrusion under the influence of tensile forces is reduced. The provision of the profile, and consequently the walls of the profile which constitute the contact surface, from said materials offers the advantage that during creation of the protrusion, for example during deep-drawing of the protrusion, the protrusion walls become thinner and more flexible in order to be able to form a rivet connection. The provision of walls from a stiffer material leads to protrusions with relatively rigid protrusion walls, which makes the formation of a rivet connection difficult. The provision of walls from a more flexible material leads to a protrusion with a relatively flexible protrusion head, which makes the forming of a rivet connection difficult.

Described is a use of the immobilisation element. More specifically, the present invention provides the initial use of the immobilisation element, wherein
- the immobilisation element is heated,
- the sheet of thermoplastic material is drawn over the body part to be immobilised, and
- after cooling of the sheet of thermoplastic material, this retains the form of the body part to be immobilised. During the initial usage of the immobilisation element according to the present invention, tensile forces occur between the sheet and the profile, which can be optimally absorbed by means of the immobilisation element of the present invention.

Described is the use of the immobilisation element furthermore comprises that, after cooling of the sheet of thermoplastic material, the immobilisation element is used for immobilising the body part of a patient on a supporting surface. During use of the immobilisation element according to the present invention, tensile forces occur between the sheet and the profile, for example by movement of the patient, which can be optimally absorbed by means of the immobilisation element of the present invention. Alternatively, the use of the immobilisation element comprises use of the immobilisation element for medical diagnoses and/or radiation therapy, for example photon therapy, proton therapy, 3-D conformal radiation therapy, IMRT (intensity-modulated radiation therapy), IGRT (image-guided radiation therapy), or stereotactic radiation therapy. In these embodiments, the patient's body part must be immobilised on the supporting surface particularly precisely, and this immobilisation is often subject to repeated positioning of the body part to be immobilised throughout the course of a treatment with little variation of the immobilisation position. It is known for example that the success of the treatment in proton radiation therapy is influenced even on a deviation of a few millimetres in the positioning of the body part between the simulation and the actual treatment, or between repeated actual treatments. Thus a deviation of a few millimetres may for example lead to irradiating not the tumour but the surrounding healthy tissue, which is unacceptable. In one embodiment of the present invention, the immobilisation element is used as an immobilisation mask for immobilising the patient's head on the supporting surface, for example for irradiating or imaging cranial indications such as a brain tumour.

Alternatively provided is a machine for performing the method of the present invention. Alternatively, the machine comprises:
- a holding device for receiving the profile and the sheet,
- a die provided with teeth for pressing at least one protrusion into the profile under the influence of a pressure force, and
- a pressing device for compressing the profile and the die so that the profile deforms plastically into the first and second contact surface provided with the at least one protrusion.

The provision of a machine for performing the method of the present invention offers the advantage that a reproducible result can be obtained because each profile is compressed in the same way with the same die.

Alternatively, the die is provided with movable teeth which, under the influence of the pressing device, are pressed out of the die and into the profile until a protrusion is created with the desired protrusion height h1. In an alternative embodiment of the present invention, the die is provided with fixed teeth which are pressed against the profile under the influence of the pressing device.

Alternatively, the holding device is provided for clamping the profile provided with the sheet in the profile groove. The holding device is provided for clamping the profile during compression of the profile and the die by the pressing device, so that the profile does not move during compression. Furthermore, the holding device is provided for clamping the profile during retraction of the teeth of the die after compression of the profile and the die, so that the profile does not move under the influence of the retracting teeth of the die.

Alternatively, the die provided in the machine is replaceable. The replaceability of the die offers the advantage that the machine can easily be adapted with another die, for example in the case of wear on the die. It is for example also possible that if a different form or arrangement of protrusions is desired for a specific type of profile and sheet, it is simple to install another die with other teeth in the machine.

### Brief Description of the Drawings

The invention will be explained in more detail below with reference to the following description and the attached drawings.
Figure 1 shows a perspective view of part of the immobilisation element.
Figure 2 shows the immobilisation element for immobilising a body part of a patient on a supporting surface.
Figure 3 shows the step of the method in which a profile and a sheet are applied.
Figure 4 shows the step of placing the sheet of thermoplastic material in the groove of the first fixing means of the profile.
Figure 5 shows a step of providing a machine for creating the protrusion in the profile.
Figure 6 shows the use of the machine during creation of the protrusion in the profile.
Figure 7 shows the immobilisation element after removal of machine for creating the protrusion.

### Embodiments of the Invention

The present invention will be described below with reference to clearly defined embodiments and with reference to specific drawings, but the invention is not restricted thereto and is defined solely by the claims. The drawings shown here are merely diagrammatic depictions and are not restrictive. In the drawings, the dimensions of certain components may be shown enlarged, which means that the components in question are not shown to scale, purely for illustrative purposes. The dimensions and relative dimensions do not necessarily correspond to actual practical embodiments of the invention.

In addition, terms such as "first", "second", "third" and similar in the description and in the claims are used to make a distinction between similar elements, and not necessarily to indicate a sequential or chronological order. The terms concerned are mutually interchangeable under appropriate circumstances, and the embodiments of the invention may function in different orders from those described or illustrated here.

The term "comprising" and derived terms as used in the claims should not be interpreted as being restricted to the means which are specified thereafter; this term does not exclude other elements or steps. The term should be interpreted as a specification of the indicated properties, numbers, steps or components to which reference is made, without excluding the presence or addition of one or more secondary properties, numbers, steps or components or groups thereof. The scope of an expression such as "a device comprising means A and B" is then not simply limited to devices which consist solely of components A and B. In contrast, it means that with regard to the present invention, the only relevant components are A and B.

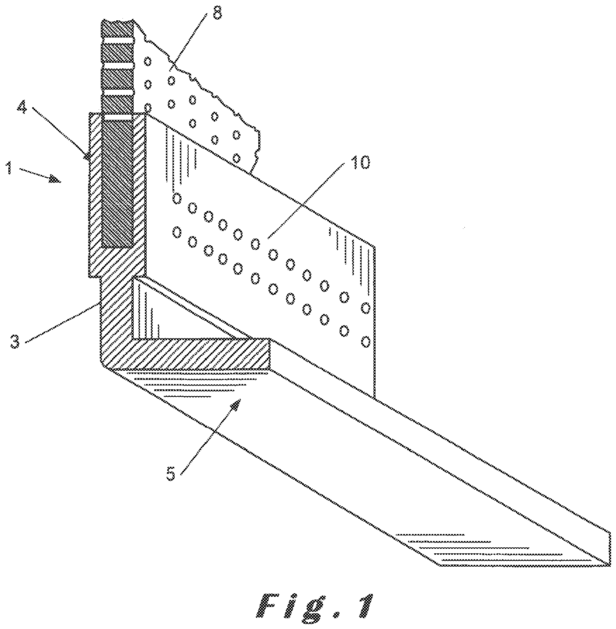

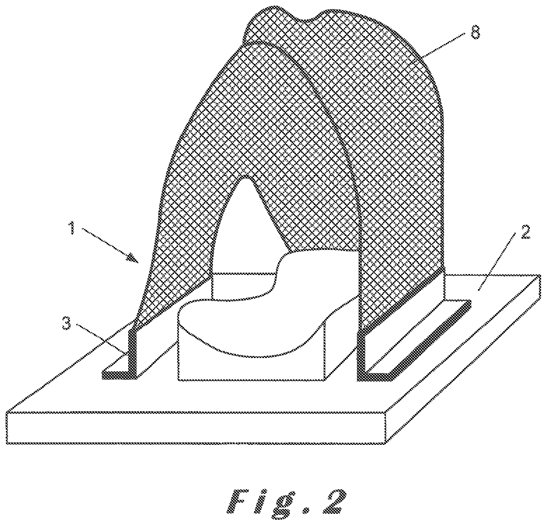

Figure 1 shows a perspective view of part of the immobilisation element 1. The immobilisation element 1 comprises a sheet 8 of thermoplastic material and a profile 3. The immobilisation element 1 is intended for immobilising a body part of a patient, such as a patient's head, on a supporting surface 2 as shown in figure 2. The sheet 8 is intended for receiving the body part to be immobilised. This is achieved by initially heating the sheet 8, for example in a warm water bath or an oven, or for example by electromagnetic radiation, so that the sheet 8 becomes deformable and can be drawn over the body part to be immobilised. After cooling of the sheet 8, and for example further hardening of the sheet, the sheet 8 is hard and rigid, which allows immobilisation of the body part during the actual treatment. For this, during the actual treatment, the body part to be immobilised is placed on the supporting surface 2, and the immobilisation element 1 is connected by means of the profile 3 to the supporting surface 2 while the already initially deformed sheet 8 is placed over the body part to be immobilised. As shown in figure 1, the profile 3 for this comprises on the first side a first fixing means 4 for fixing the profile 3 to the sheet 8, and the profile 3 furthermore comprises, on a second side opposite the first side, a second fixing means 5 for releasably fixing the profile 3 to the supporting surface 2. Since the second fixing means 5 attaches the profile 3 releasably to the supporting surface 2, the immobilisation element 1 may be used for repeated actual treatments of the patient, preferably the same patient. The immobilisation elements 1 as shown in figures 1 and 2 are often used for medical diagnoses and/or radiation therapy, wherein the immobilisation, for example the repeated immobilisation, of the patient's body part during diagnosis or treatment is of crucial importance. The firm and durable fixing of the sheet 8 to the profile 3, for example during repeated treatments, despite the high tensile forces which occur between the sheet 8 and the profile 3 during the initial deformation of the sheet 8 and during the actual use of the immobilisation element 1 in a treatment, is achieved by anchoring the sheet 8 to the profile 3 by means of at least one protrusion 10 which penetrates the sheet 8. The immobilisation element 1 as shown in figure 1 is provided with a set of protrusions 10 which penetrate the sheet 8 in order to attach the sheet 8 to the profile 3.

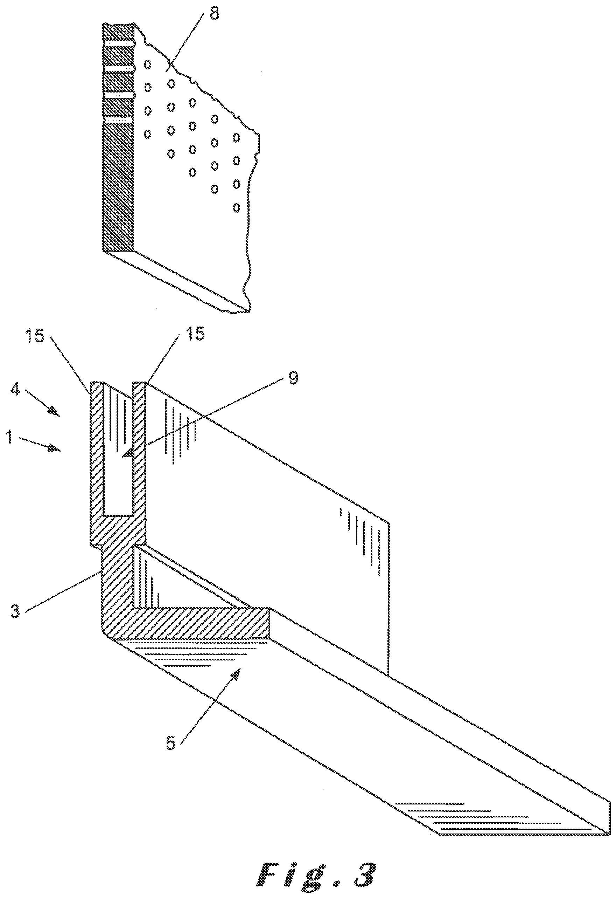

The method for producing the immobilisation element 1 is discussed further with reference to figures 3 to 7. The method for producing the immobilisation element 1 provides a number of successive steps which may be performed either by the end user of the immobilisation element 1, for example by the nursing personnel before initial deformation of the sheet 8, or by the supplier of the immobilisation element 1 before delivery of the immobilisation element 1 to the end user.

The method for producing the immobilisation element 1 comprises as a first step the provision of a profile 3 as shown in figure 3. The profile which is provided comprises on a first side a first fixing means 4 for fixing the profile 3 to the sheet 8, and on a second side opposite the first side a second fixing means 5 for releasably fixing the profile 3 to the supporting surface 2. The user of the immobilisation element 1 provides a profile 3 which is provided with the correct type of second fixing means 5 corresponding to the supporting surface 2 of the user and the desired fixing of the profile to the supporting surface 2. The first fixing means 4 of the profile 3 comprises a groove 9 delimited by two opposite groove walls 15 which are integrally connected to each other. The groove 9 has an elongate direction parallel to the supporting surface 2, which is referred to as the longitudinal direction of the groove 9, a shorter transverse direction approximately perpendicular to the opposing groove walls 15, and a depth direction perpendicular to the longitudinal direction and the transverse direction of the groove 9. The entire profile 3 is made of one piece, in other words the first fixing means 4 is integrally connected to the second fixing means 5. Figure 3 also shows the step of providing a sheet 8 of thermoplastic material. The user of the immobilisation element 1 provides a sheet 8 which has the correct properties, such as a suitable sheet size and thickness, a suitable material and a suitable form corresponding to the expected use of the immobilisation element 1. Thus the user for example provides a larger and thicker sheet 8 for an obese person compared with a non-obese person, for immobilising the same body part, since the provision of a sheet 8 which is too small and too thin for an obese person leads to overstretching of the sheet 8 in the initial deformation of the sheet, which increases the risk of failure of the immobilisation element 1. Thus the user for example also provides a sheet 8, the shape of which is adapted to the body part to be immobilised, for example a shape adapted for immobilising the patient's head as shown in figure 2. The sheet 8 of thermoplastic material as shown in figure 3 is perforated, for example to allow evaporation via the skin, or to allow visual inspection of the skin under the sheet, or to reduce attenuation of the sheet 8 to a minimum. The part of the sheet 8 of thermoplastic material which is intended to be placed in the groove 9 of the profile 3 is not perforated, in order to the reduce the risk of deformation or tearing of the sheet 8 in the groove 9 during use of the immobilisation element 1.

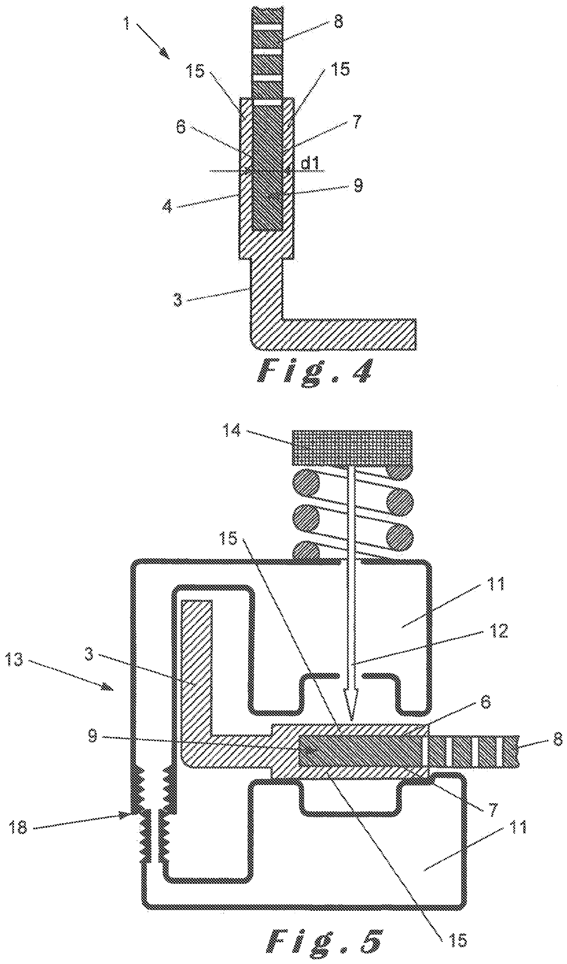

Figure 4 shows the step of placing the sheet 8 of thermoplastic material in the groove 9 of the first fixing means 4. Figure 4 shows the arranged profile 3 in a cross-section perpendicular to the longitudinal direction of the groove 9. The groove 9 of the first fixing means 4 receives the sheet 8 of thermoplastic material. The opposing groove walls 15 respectively describe a first contact surface 6 and a second contact surface 7 opposite the first contact surface 6, as the part of the groove walls 15 which lie along the sheet 8. The sheet 8 has a sheet thickness d1 approximately equal to the width of the groove 9.

In order to anchor the sheet 8 firmly and durably in the groove 9 of the profile 3, a set of protrusions must be created in the contact surface 6, 7 of the profile 3 such that the protrusions penetrate the sheet 8. Figures 5 to 7 show an exemplary embodiment for creating a protrusion. As shown in figure 5, a machine is provided for this. The machine comprises a holding device 13, for example a holder, for receiving the profile 3 and the sheet 8. The machine furthermore comprises two dies 11 provided with teeth 12 for pressing the at least one protrusion into the contact surface of the profile 3 under the influence of a pressure force. In figure 5, the two dies 11 lie adjacent to the groove walls 15. Alternatively, only one die may be provided, lying adjacent to one of the groove walls, in order to provide protrusions only in one groove wall 15. Figure 5 furthermore shows only one die 11 with teeth 12, wherein figure 5 shows a transverse cross-section of the machine, and the teeth 12 in the machine shown are aligned in the longitudinal direction on the one die 11 relative to the opposite die 11. The machine furthermore comprises a pressing device 14 for compressing the profile 3 and the teeth 12 of the die 11, so that the profile 3 deforms plastically until the first and second contact surfaces 6, 7 are provided with at least one protrusion. In order to press the teeth 12 and the profile 3 together, firstly the teeth 12 are provided movably in the die 11, wherein the pressing device 14 pushes the teeth 12 out of the die 11, or secondly the teeth 12 are connected integrally with the die 11, wherein the pressing device 14 presses the complete die 11 provided with teeth 12 against the profile 3. Figure 5 shows a die 11 with movable teeth 12. The transverse cross-section shown in figure 5 depicts only one tooth 12 in the depth direction of the groove 9. Preferably, the machine comprises a set of teeth 12 which correspond to the pattern of protrusions to be applied, for example by providing several teeth 12 in the depth direction of the groove 9. The holding device 13 is furthermore provided with an adjustment device 18 provided for bringing the dies 11 towards each other or moving them apart. If the dies 11 are brought towards each other, this ensures amongst others that the profile 3 is fixedly held during creation of the protrusion and during retraction of the teeth 12 from the protrusion.

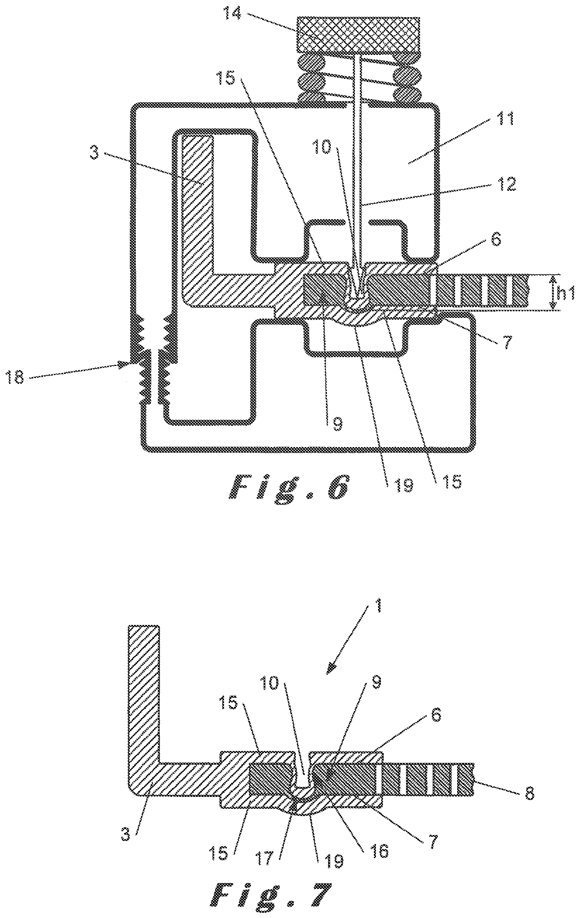

Figure 6 shows the use of the machine during creation of the protrusion 10 in the profile 3. More specifically, figure 6 shows the teeth 12 of the die 11 which plastically deform the contact surface 6 of the profile 3 in order to form a protrusion 10. The teeth 12 of the die 11 penetrate the sheet 8 maximally. Here the sheet 8 is not perforated but merely partially penetrated. In one embodiment (not illustrated in figure 6), the formed protrusion 10 protrudes maximally by a height h1 from the contact surface 6 on which the protrusion is provided, and the height h1 is smaller than the width of the groove, which for example is equal to the thickness d1 of the sheet 8, such that the protrusion cannot bore through the sheet, for example cannot penetrate it completely. Part of the sheet 8 is consequently clamped between the protrusion 10 and the contact surface 7 opposite the contact surface 6 on which the protrusion 10 is provided. The clamped sheet 8 forms a fleece which ensures a firm adhesion of the sheet 8 to the protrusion 10 and the contact surface 7 opposite the contact surface 6 on which the protrusion 10 is provided. It is also possible that on maximal penetration of the teeth 12 into the profile 3, the height h1 is greater than the width of the groove, which for example is equal to the thickness d1 of the sheet 8, as long as the sheet 8 is only partially penetrated in order to form a fleece. This situation is shown in figure 6. Figure 6 furthermore shows that the contact surface 7 opposite the contact surface 6 on which the at least one protrusion 10 is provided, opposite the at least one protrusion 10, is provided with at least one bulge 19, wherein the bulge 19 points away from the sheet 8. More specifically, the bulge 19 is oriented in the same direction as the opposite protrusion 10. The clamp sheet 8 forms a fleece which ensures a firm adhesion of the sheet 8 to the protrusion 10 and the bulge 19.

Figure 7 shows the immobilisation element 1 after removal of the machine for creating the protrusion 10. When the teeth 12 of the machine have been removed from the profile 3 and the sheet 8, a hollow protrusion 10 remains. In the example described here, the material properties and dimensions of the groove walls 15 and the sheet 8 of thermoplastic material are selected such that the created protrusion 10 forms a rivet connection. If the protrusion 10 is formed by deep-drawing of a small area of the contact surface 6, a protrusion is created with thin protrusion walls 16 and a relatively thick protrusion head 17. The resulting created protrusion 10 has a relatively hard and rigid protrusion head 17 compared with relatively flexible protrusion walls 16. As shown in figure 6, the sheet 8 of thermoplastic material is elastically stretched by penetration of the teeth 12, whereby the sheet 8 tends to spring back resiliently after removal of the teeth 12. Since the protrusion walls 16 are relatively flexible compared with the protrusion head 17, the sheet 8 springs back elastically by compression of the protrusion walls 16, while the protrusion head 17 remains almost unchanged. Here, at least part of the sheet 8 is pinched between the protrusion head 17 and the contact surface 6 on which the protrusion 10 is formed, i.e. a rivet connection is formed. The rivet connection thus formed ensures an optimal anchoring of the sheet 8 in the groove 9 of the profile 3. The anchoring provides a firm and durable connection of the sheet without the need to use external materials to form the rivet connection. In addition, the rivet connection thus formed is almost unaffected by atmospheric influences, since no secondary adhesive substances must be used such as glue.

## Claims

1. Method for producing an immobilisation element (1) for immobilising a body part of a patient on a supporting surface (2), wherein the method comprises the following steps:
• providing a sheet (8) of thermoplastic material for receiving the body part to be immobilised,
• providing a profile (3) which on a first side comprises a first fixing means (4) for fixing the profile (3) to the sheet (8), and on a second side different from the first side comprises a second fixing means (5) for releasably fixing the profile (3) to the supporting surface (2), wherein the first fixing means (4) comprises a groove (9) delimited by a first contact surface (6) and a second contact surface (7) opposite the first contact surface (6) for receiving the sheet (8) of thermoplastic material,
• placing the sheet (8) of thermoplastic material in the groove (9) of the first fixing means (4) so that the first and the second contact surfaces (6, 7) lie along the sheet (8),
• creating at least one protrusion (10) in at least one of the first contact surface (6) and the second contact surface (7) so that the protrusion (10) penetrates the sheet (8) of thermoplastic material in order to anchor the sheet (8) of thermoplastic material in the groove (9), wherein the step of creating at least one protrusion (10) in at least one of the first contact surface (6) and second contact surface (7) is carried out by compressing a die (11) and the profile (3) so that the profile (3) is plastically deformed into the first and second contact surfaces (6, 7) provided with at least one protrusion (10).

2. Method for producing the immobilisation element (1) according to the preceding claim, wherein the steps are successive steps.

3. Method for producing the immobilisation element (1) according to any one of the preceding claims, wherein the die (11) is provided with teeth (12) which press the at least one protrusion (10) into the profile (3).

4. Immobilisation element (1) obtainable by performing the method according to any one of the preceding claims 1-3.

## Patentansprüche

1. Verfahren zur Herstellung eines Immobilisierungselements (1) zum Immobilisieren eines Körperteils eines Patienten auf einer Auflagefläche (2), wobei das Verfahren die folgenden Schritte umfasst:
• Bereitstellen einer Platte (8) aus thermoplastischem Material zum Aufnehmen des zu immobilisierenden Körperteils,
• Bereitstellen eines Profils (3), welches an einer ersten Seite ein erstes Befestigungsmittel (4) zum Befestigen des Profils (3) an der Platte (8) umfasst, und an einer zweiten, von der ersten Seite unterschiedlichen Seite ein zweites Befestigungsmittel (5) zum lösbaren Befestigen des Profils (3) an der Auflagefläche (2) umfasst, wobei das erste Befestigungsmittel (4) eine Nut (9) umfasst, begrenzt durch eine erste Kontaktfläche (6) und eine zweite Kontaktfläche (7) gegenüber der ersten Kontaktfläche (6), um die Platte (8) aus thermoplastischem Material aufzunehmen,
• Einlegen der Platte (8) aus thermoplastischem Material in die Nut (9) des ersten Befestigungsmittels (4), sodass die erste und die zweite Kontaktfläche (6, 7) entlang der Platte (8) liegen,
• Erzeugen von zumindest einem Vorsprung (10) in zumindest einer der ersten Kontaktfläche (6) und zweiten Kontaktfläche (7), sodass der Vorsprung (10) die Platte (8) aus thermoplastischem Material durchbohrt, um die Platte (8) aus thermoplastischem Material in der Nut (9) zu verankern, wobei der Schritt des Erzeugens von zumindest einem Vorsprung (10) in zumindest einer der ersten Kontaktfläche (6) und zweiten Kontaktfläche (7) durch Zusammendrücken einer Stanze (11) und des Profils (3) durchgeführt wird, sodass das Profil (3) plastisch in die erste und die zweite Kontaktfläche (6, 7) versehen mit zumindest einem Vorsprung (10) verformt wird.

2. Verfahren zur Herstellung des Immobilisierungselements (1) nach dem vorigen Anspruch, wobei die Schritte aufeinanderfolgende Schritte sind.

3. Verfahren zur Herstellung des Immobilisierungselements (1) nach irgendeinem der vorigen Ansprüche, wobei die Stanze (11) mit Zähnen (12) versehen ist, welche den zumindest einen Vorsprung (10) in das Profil (3) drücken.

4. Immobilisierungselement (1), erhältlich durch Ausführen des Verfahrens nach irgendeinem der vorigen Ansprüche 1 bis 3.

## Revendications

1. Procédé de production d'un élément d'immobilisation (1) pour immobiliser une partie de corps d'un patient sur une surface de support (2), dans lequel le procédé comprend les étapes suivantes :
• la fourniture d'une feuille (8) de matériau thermoplastique pour recevoir la partie de corps à immobiliser,
• la fourniture d'un profilé (3) qui, d'un premier côté, comprend un premier moyen de fixation (4) pour fixer le profilé (3) à la feuille (8) et, d'un second côté différent du premier côté, comprend un second moyen de fixation (5) pour fixer de manière libérable le profilé (3) à la surface de support (2), dans lequel le premier moyen de fixation (4) comprend une rainure (9) délimitée par une première surface de contact (6) et une seconde surface de contact (7) opposée à la première surface de contact (6) pour recevoir la feuille (8) de matériau thermoplastique,
• le positionnement de la feuille (8) de matériau thermoplastique dans la rainure (9) du premier moyen de fixation (4) de sorte que les première et seconde surfaces de contact (6, 7) s'étendent le long de la feuille (8),
• la création d'au moins une saillie (10) dans au moins l'une de la première surface de contact (6) et de la seconde surface de contact (7) de sorte que la saillie (10) pénètre dans la feuille (8) de matériau thermoplastique afin d'ancrer la feuille (8) de matériau thermoplastique dans la rainure (9), dans lequel l'étape de création d'au moins une saillie (10) dans au moins l'une de la première surface de contact (6) et de la second surface de contact (7) est mise en oeuvre par compression d'une matrice (11) et du profilé (3) de sorte que le profilé (3) soit déformé plastiquement dans les première et seconde surfaces de contact (6, 7) dotées d'au moins une saillie (10).

2. Procédé de production de l'élément d'immobilisation (1) selon la revendication précédente, dans lequel les étapes sont des étapes successives.

3. Procédé de production de l'élément d'immobilisation (1) selon l'une quelconque des revendications précédentes, dans lequel la matrice (11) est dotée de dents (12) qui pressent la au moins une saillie (10) dans le profilé (3).

4. Élément d'immobilisation (1) pouvant être obtenu par réalisation du procédé selon l'une quelconque des revendications 1-3 précédentes.
